# EUROPÄISCHE PATENTSCHRIFT

(11) **EP 0 865 500 B1**
(45) Veröffentlichungstag und Bekanntmachung des Hinweises auf die Patenterteilung: **13.02.2002**
(21) Anmeldenummer: 96939915.3
(22) Anmeldetag: 25.11.1996
(51) Int. Cl.: C12P 41/00, C07C 235/06

(54) **VERFAHREN ZUR HERSTELLUNG VON OPTISCH AKTIVEN AMINEN**
PROCESS FOR THE PREPARATION OF OPTICALLY ACTIVE AMINES
PROCEDE DE PRODUCTION D'AMINES OPTIQUEMENT ACTIVES

(30) Priorität: 06.12.1995 DE 19545466; 13.09.1996 DE 19637336
(43) Veröffentlichungstag der Anmeldung: 23.09.1998
(73) Patentinhaber: BAYER AG, 51368 Leverkusen (DE)
(72) Erfinder: STELZER, Uwe, D-51399 Burscheid (DE); DREISBACH, Claus, D-51065 Köln (DE)
(86) Internationale Anmeldenummer: EP9605188
(87) Internationale Veröffentlichungsnummer: WO9720946

(56) Entgegenhaltungen:
- WO-A-97/10201
- DE-A- 4 332 738
- DE-A- 19 523 151
- CHIMIA, Bd. 48, Nr. 12, Dezember 1994, Seite 570 XP000645837 MANFRED T. REETZ ET AL.: "Highly efficient lipase-catalyzed kinetic resolution of chiral amines" in der Anmeldung erwähnt

## Beschreibung

Die vorliegende Erfindung betrifft ein neues Verfahren zur Herstellung von bekannten, optisch aktiven Aminen, die als Zwischenprodukte zur Herstellung von Pharmazeutika und Pflanzenschutzmitteln verwendet werden können. Die Erfindung betrifft außerdem neue optisch aktive acylierte Amine.

Aus der DE-A 4 332 738 ist bereits bekannt, daß sich optisch aktive, primäre und sekundäre Amine herstellen lassen, indem man zunächst racemisches Amin in Gegenwart einer Hydrolase mit einem Ester, der im Säureteil in Nachbarschaft des Carbonylkohlenstoffatoms ein elektronenreiches Heteroatom aufweist, enantioselektiv acyliert, dann das entstehende Gemisch aus optisch aktivem (S)-Amin und optisch aktivem acylierten (R)-Amin (= Amid) trennt, dadurch das (S)-Amin erhält und gegebenenfalls aus dem acylierten (R)-Amin durch Amid-Spaltung das andere Enantiomere gewinnt. Als Hydrolasen kommen dabei Lipasen aus Pseudomonas, z.B. Amano P, oder aus Pseudomonas spec. DSM 8246 in Frage. Der optische Reinheitsgrad der anfallenden Enantiomeren ist sehr hoch. Nachteilig an diesem Verfahren ist aber, daß bei der enzymatischen Acylierung recht lange Reaktionszeiten erforderlich sind und in stark verdünnter Lösung gearbeitet wird. Das verbleibende (S)-Enantiomer wird erst nach relativ langen Reaktionszeiten in ausreichend hoher optischer Ausbeute erhalten. Die erzielbaren Raum-Zeit-Ausbeuten lassen daher für praktische Zwecke zu wünschen übrig. Ungünstig ist auch, daß in Bezug auf das Substrat verhältnismäßig hohe Mengen an Enzym erforderlich sind. Im übrigen weist das Enzym eine sehr hohe Aktivität auf, so daß ein erheblicher Aufwand für die Reinigung, die Konzentrierung und die Aufarbeitung notwendig ist.

Weiterhin geht aus Chimia 48, 570 (1994) hervor, daß racemische Amine mit Essigsäureethylester in Gegenwart von Lipase aus Candida antarctica enantioselektiv zu Gemischen aus (S)-Amin und acetyliertem (R)-Amin (= Amid) reagieren, aus denen (S)-Amin und acetyliertes (R)-Amin isoliert werden können,
wobei das acetylierte (R)-Amin durch anschließende Amid-Spaltung freigesetzt werden kann. Ungünstig an dieser Methode ist, daß wiederum recht lange Reaktionszeiten benötigt werden und außerdem die Ausbeuten nicht immer befriedigend sind. Weiterhin ist auch hierbei das Verhältnis von Enzym zu Substrat so unvorteilhaft, daß eine wirtschaftliche Nutzung des Verfahrens kaum möglich ist.

Im übrigen ist aus der vorgängigen, aber nicht vorveröffentlichten Patentanmeldung gemäß DE - A 195 34 208 (= WO 97 - 10 201 ) bekannt,daß sich optisch aktive Arylalkylamine herstellen lassen , indem man die entsprechenden Racemate mit bestimmten Estern in Gegenwart von Lipasen, wie Candida antarctica, umsetzt. Es werden aber keine Phenylalkylamine erwähnt, in denen der Phenylring in den Positionen 3, 4 und / oder 5 durch bestimmte Reste substituiert ist.

Es wurde nun gefunden, daß man optisch aktive Amine der Formel 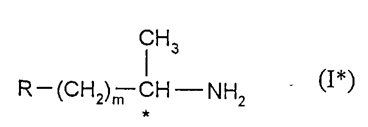 in welcher
- R: für substituiertes Phenyl der Formel 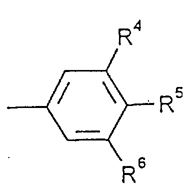 steht, worin
- R⁴, R⁵ und R⁶: unabhängig voneinander für Wasserstoff, Fluor, Chlor, Brom, Methyl, Ethyl, n-Propyl, Isopropyl, n-Butyl, iso-Butyl, sek.-Butyl, Methoxy, Ethoxy, Methylthio, Trichlormethyl, Trifluormethyl, Difluormethyl, Trifluormethoxy, Difluorchlormethoxy, Difluormethoxy, Cyano, Dimethylamino, Diethylamino, Nitro, Phenyl, Phenoxy oder Benzyl stehen,
wobei aber mindestens einer der Reste
R⁴, R⁵ und R⁶ nicht für Wasserstoff steht,
oder
- R: für gegebenenfalls einfach bis dreifach, gleichartig oder verschieden durch Fluor, Chlor, Brom, Methyl, Ethyl, n-Propyl, Isopropyl, n-Butyl, iso-Butyl, sek.-Butyl, Methoxy, Ethoxy, Trichlormethyl, Trifluormethyl, Difluormethyl, Trifluormethoxy, Difluorchlormethoxy und/oder Difluormethoxy substituiertes Naphthyl steht, wobei aber die ortho-Positionen zu dem Kohlenstoffatom, über das der Naphthyl-Rest gebunden ist, nicht substituiert sind,
oder
- R: für gegebenenfalls benzanelliertes Furyl, Thienyl, Pyridyl oder Pyrimidin steht, wobei diese Reste einfach bis dreifach, gleichartig oder verschieden substituiert sein können durch Fluor, Chlor, Brom, Methyl, Ethyl, n-Propyl, Isopropyl, n-Butyl, iso-Butyl, sek.-Butyl, tert.-Butyl, Methoxy, Ethoxy, n-Propoxy, iso-Propoxy, Trifluormethyl und/oder Trifluorethyl, wobei aber die zur Verknüpfungsstelle benachbarten Positionen der Heteroarylgruppe keine Substituenten tragen,
oder
- R: für geradkettiges oder verzweigtes Alkyl mit 1 bis 5 Kohlenstoffatomen oder Halogenalkyl mit 1 bis 5 Kohlenstoffatomen und 1 bis 3 Fluor- und/oder Chloratomen steht,
und
- m: für die Zahlen 0, 1 oder 2 steht,
dadurch gekennzeichnet, daß man
a) in einer ersten Stufe racemische Amine der Formel 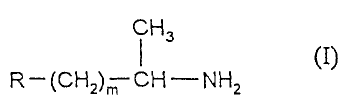 in welcher
   R und m die oben angegebenen Bedeutungen haben,
   mit Estern der Formel 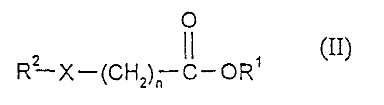 in welcher
   - R¹: für Methyl, Ethyl, n-Propyl, n-Butyl, Chlormethyl, 2-Chlorethyl, 2-Fluorethyl oder 2,2,2-Trifluorethyl steht,
   - R²: für Wasserstoff, Methyl, Ethyl, n-Propyl, n-Butyl, Chlormethyl, Trifluormethyl, 2-Chlorethyl oder für gegebenenfalls einfach oder zweifach durch Fluor, Chlor, Brom, Amino, Hydroxy, Methyl, Ethyl, Methoxy, Phenyl und/oder Phenoxy substituiertes Phenyl steht,
   - X: für Sauerstoff oder Schwefel steht und
   - n: für die Zahlen 1 oder 2 steht;
   in Gegenwart von Lipase aus Candida antarctica sowie gegebenenfalls in Gegenwart eines Verdünnungsmittels umsetzt,
b) in einer zweiten Stufe das erhaltene Gemisch aus (S)-Amin der Formel 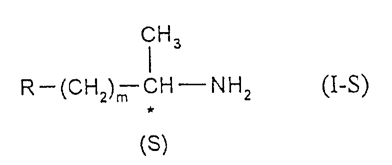 in welcher
   R und m die oben angegebenen Bedeutungen haben,
   und acyliertem (R)-Amin der Formel 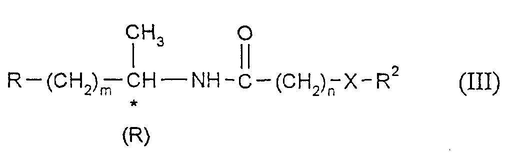 in welcher
   R, R², X, m und n die oben angegebenen Bedeutungen haben,
   trennt und
c) gegebenenfalls in einer dritten Stufe aus acyliertem (R)-Amin der Formel (III) durch Behandlung mit Säure oder Base, gegebenenfalls in Gegenwart eines Verdünnungsmittels (R)-Amin der Formel 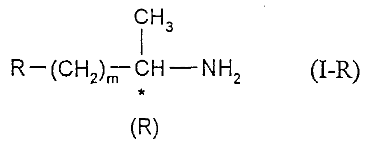 in welcher
   R und m die oben angegebenen Bedeutungen haben,
   freisetzt.

Unter (R)-Aminen sind diejenigen optisch aktiven Verbindungen der Formel (I) zu verstehen, die am asymmetrisch substituierten Kohlenstoffatom die (R)-Konfiguration aufweisen. Entsprechend sind unter (S)-Aminen diejenigen optisch aktiven Verbindungen der Formel (I) zu verstehen, die am Chiralitätszentrum die (S)-Konfiguration aufweisen. In den Formeln ist das asymmetrisch substituierte Kohlenstoffatom jeweils durch (*) gekennzeichnet.

Es ist als äußerst überraschend zu bezeichnen, daß sich optisch aktive Amine der Formel (I*) nach dem erfindungsgemäßen Verfahren in hoher Ausbeute und sehr guter optischer Reinheit herstellen lassen. Aufgrund des bekannten Standes der Technik konnte nämlich nicht damit gerechnet werden, daß die spezielle Verwendung von Lipase aus Candida antarctica eine höhere Enantioselektivität und eine schnellere Reaktion bei der Umsetzung zwischen Amin und Ester bewirkt als die in entsprechenden Verfahren bisher verwendeten Enzymsysteme.

Das erfindungsgemäße Verfahren weist eine Reihe von Vorteilen auf. So ermöglicht es die Herstellung einer Vielzahl von optisch aktiven Aminen in hoher Ausbeute und hervorragender optischer Reinheit. Günstig ist auch, daß bei relativ hoher Substrat-Konzentration gearbeitet werden kann und die Reaktionszeiten kurz sind. Dadurch lassen sich Raum-Zeit-Ausbeuten erzielen, die auch für praktische Zwecke befriedigend sind. Von Vorteil ist auch, daß der benötigte Biokatalysator in größerer Menge zur Verfügung steht und auch bei erhöhter Temperatur stabil ist. Dabei wird der Biokatalysator bezüglich der Enzym-Menge im Verhältnis zum Substrat in relativ geringer Menge und niedriger Enzymaktivität eingesetzt. Schließlich bereitet auch die Durchführung der Umsetzung und die Isolierung der gewünschten Substanzen, und zwar sowohl der (S)- als auch der (R)-Amine, keinerlei Schwierigkeiten.

Setzt man racemisches 1-(4-Chlorphenyl)-ethylamin in Gegenwart von Lipase aus Candida antarctica mit Methoxyessigsäuremethylester um, trennt die entstehenden Komponenten und behandelt das (R)-Enantiomere des Methoxyessigsäure-1-(4-chlorphenyl)-ethylamids mit Salzsäure, so kann der Verlauf des erfindungsgemäßen Verfahrens durch das folgende Formelschema veranschaulicht werden.

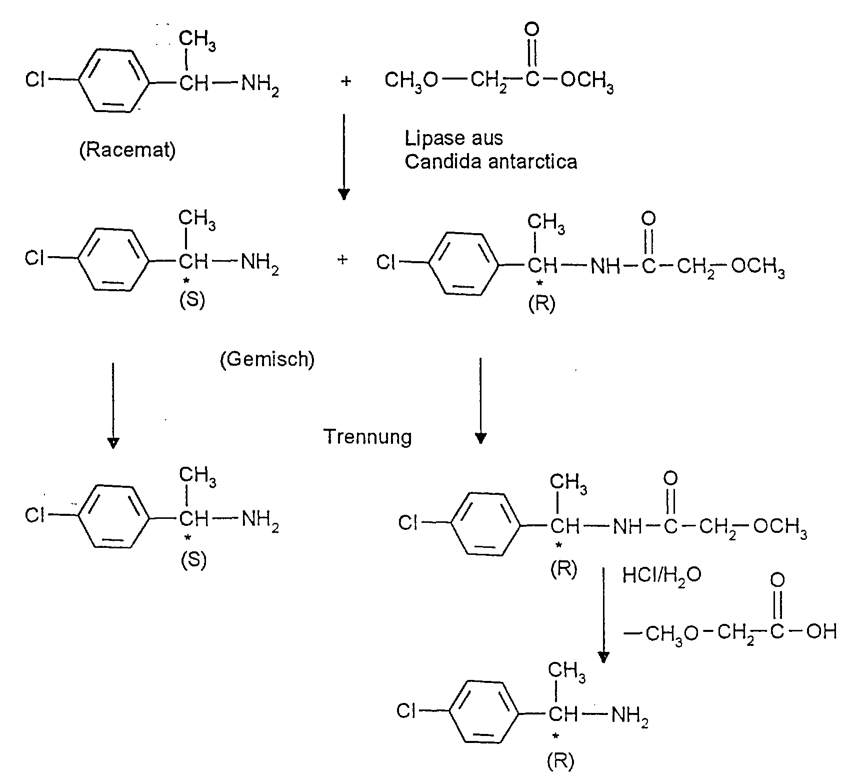

Die bei der Durchführung des erfindungsgemäßen Verfahrens als Ausgangsstoffe benötigten racemischen Amine sind durch die Formel (I) allgemein definiert.

Als Beispiele für Amine der Formel (I) seien die Verbindungen der folgenden Formeln genannt:

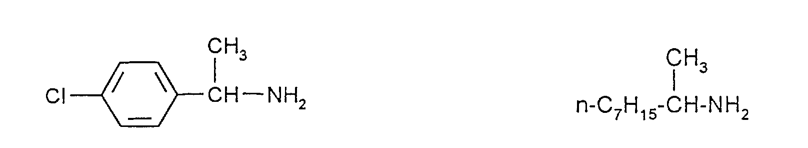

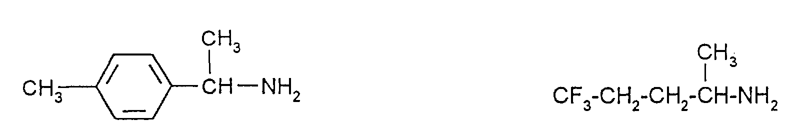

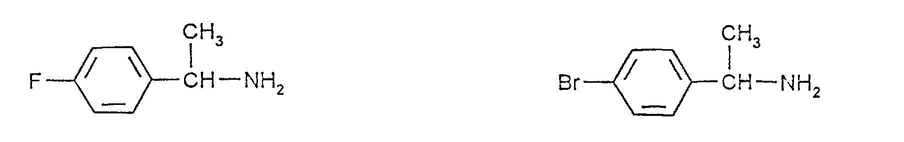

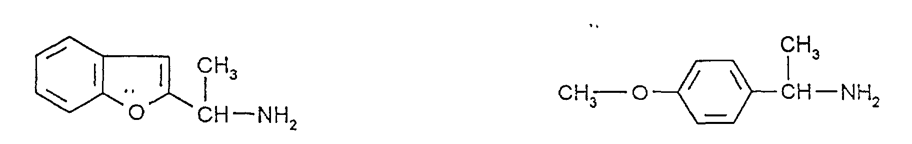

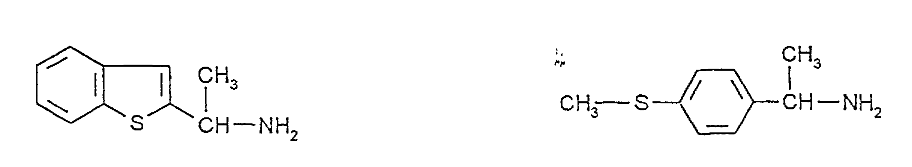

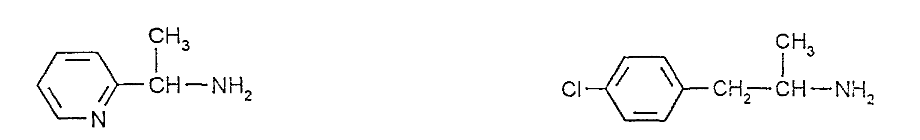

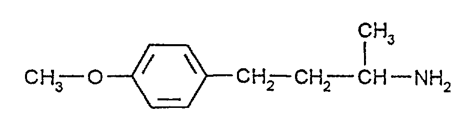

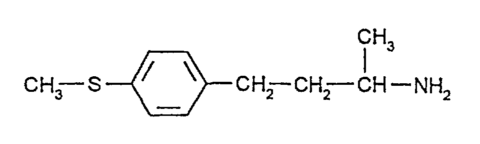

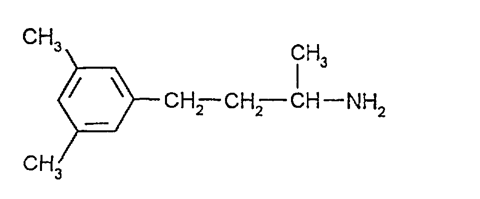

Die racemischen Amine der Formel (I) sind bekannt oder lassen sich nach bekannten Methoden herstellen.

Die bei der Durchführung der ersten Stufe des erfindungsgemäßen Verfahrens als Reaktionskomponenten benötigten Ester sind durch die Formel (II) allgemein definiert.

Als Beispiele für Ester der Formel (II) seien die Verbindungen der folgenden Formeln genannt.

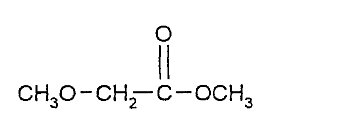

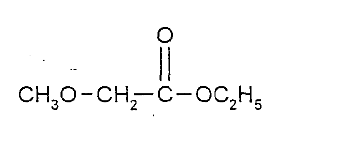

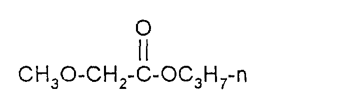

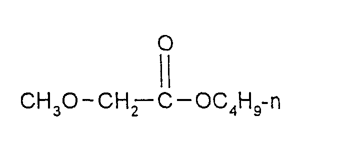

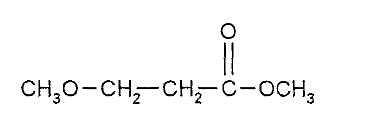

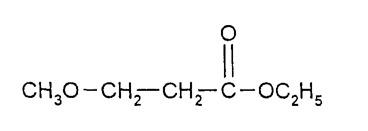

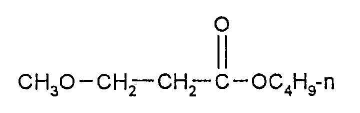

Die Ester der Formel (II) sind bekannt oder lassen sich nach bekannten Methoden herstellen.

Als Biokatalysator dient bei der Durchführung der ersten Stufe des erfindungsgemäßen Verfahrens Lipase aus Candida antarctica. Bevorzugt verwenbar ist das unter der Bezeichnung Novozym 435® kommerziell erhältliche Produkt.

Die Lipase kann entweder nativ oder in modifizierter Form, z.B. mikroverkapselt oder an anorganische oder organische Trägermaterialien gebunden, eingesetzt werden. Als Trägermaterialien kommen dabei z.B. Celite, Lewatit, Zeolithe, Polysaccharide, Polyamide und Polystyrolharze in Frage.

Als Verdünnungsmittel kommen bei der Durchführung der ersten Stufe des erfindungsgemäßen Verfahrens alle für derartige Umsetzungen üblichen organischen Solventien in Betracht. Vorzugsweise verwendbar sind Ether, wie Methyl-tert.-butyl-ether oder tert.-Amyl-methyl-ether, ferner aliphatische oder aromatische Kohlenwasserstoffe, wie Hexan, Cyclohexan oder Toluol, außerdem Nitrile, wie Acetonitril oder Butyronitril, weiterhin Alkohole, wie tert.-Butanol oder 3-Methyl-3-pentanol, und schließlich auch die zur Acylierung eingesetzten Ester.

Die Temperaturen können bei der Durchführung der ersten Stufe des erfindungsgemäßen Verfahrens innerhalb eines bestimmten Bereiches variiert werden. Im allgemeinen arbeitet man bei Temperaturen zwischen 0°C und 80°C, vorzugsweise zwischen 10°C und 60°C.

Bei der Durchführung der ersten Stufe des erfindungsgemäßen Verfahrens arbeitet man im allgemeinen unter Atmosphärendruck, gegebenenfalls unter Inertgas, wie Stickstoff oder Argon.

Bei der Durchführung der ersten Stufe des erfindungsgemäßen Verfahrens setzt man auf 1 Mol an racemischem Amin der Formel (I) im allgemeinen 0,6 bis 10 Mol, vorzugsweise 1 bis 3 Mol an Ester der Formel (II) ein. Die Menge an Lipase kann ebenfalls innerhalb eines bestimmten Bereiches variiert werden. Im allgemeinen setzt man 1 bis 10 Gew.-% an immobilisierter Lipase, bezogen auf racemisches Amin ein, was einer Aktivität von 10 000 bis 112 000 Units Lipase pro Mol an racemischem Amin entspricht. Im einzelnen verfährt man bei der Durchführung der ersten Stufe des erfindungsgemäßen Verfahrens in der Weise, daß man die Komponenten in beliebiger Reihenfolge zusammengibt und das entstehende Gemisch bis zum Erreichen des gewünschten Umsatzes bei der jeweiligen Reaktionstemperatur rührt. Zur Beendigung der Reaktion geht man im allgemeinen so vor, daß man den Biokatalysator durch Filtration abtrennt.

Das in der ersten Stufe des erfindungsgemäßen Verfahrens erhaltene Gemisch wird in der zweiten Stufe nach üblichen Methoden aufgearbeitet. Im allgemeinen isoliert man die gewünschten Komponenten durch Destillation, fraktionierte Kristallisation, Säure-Base-Lösungsmittel-Extraktion oder auf anderem Wege. So kann man zum Beispiel in der Weise verfahren, daß man das Reaktionsgemisch einer fraktionierten Destillation unterwirft. Es ist auch möglich, so vorzugehen, daß man das Reaktionsgemisch einengt, den verbleibenden Rückstand in einem mit Wasser nur wenig mischbaren, organischen Solvens aufnimmt, die entstehende Lösung mit Wasser und Mineralsäure behandelt und die Phasen trennt. Durch Einengen der organischen Phase erhält man das acylierte (R)-Amin. Aus der wäßrigen Phase läßt sich das (S)-Amin isolieren, indem man zunächst mit Base behandelt, dann mit einem mit Wasser nur wenig mischbaren organischen Solvens extrahiert, die vereinigten organischen Phasen trocknet und einengt. - Gegebenenfalls kann eine weitere Reinigung der isolierten Produkte, z.B. durch Chromatographie oder Destillation, vorgenommen werden.

Die acylierten (R)-Amine der Formel 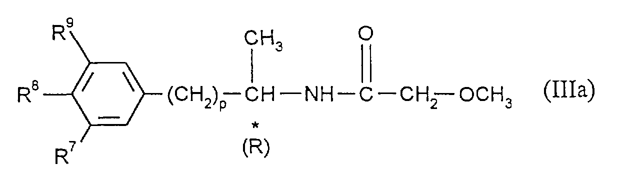 in welcher
- R⁸: für Fluor, Chlor, Brom, Methyl, Methoxy oder Methylthio steht,
- R⁷ und R⁹: für Wasserstoff stehen und
- p: für die Zahlen 0, 1 oder 2 steht,
oder
- R⁸: für Wasserstoff steht,
- R⁷ und R⁹: für Methyl stehen und
- p: für 2 steht,
sind neu.

Als Beispiele für acylierte (R)-Amine der Formel (IIIa) seien die Verbindungen der folgenden Formeln genannt:

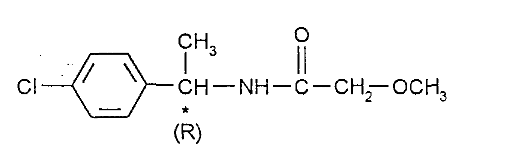

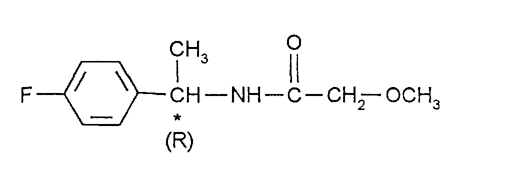

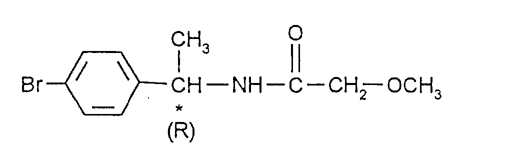

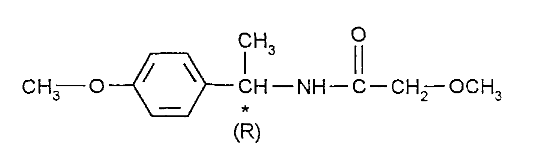

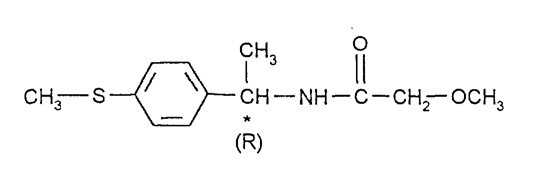

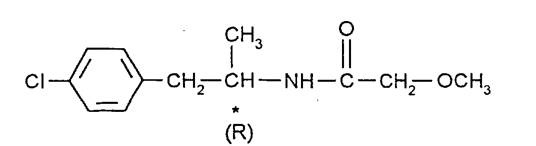

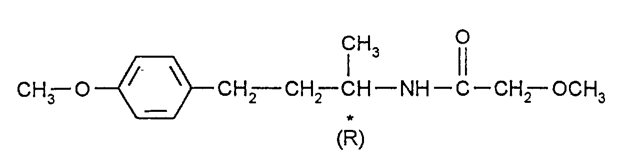

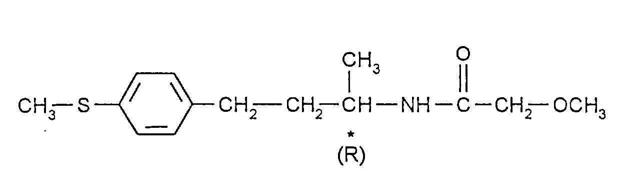

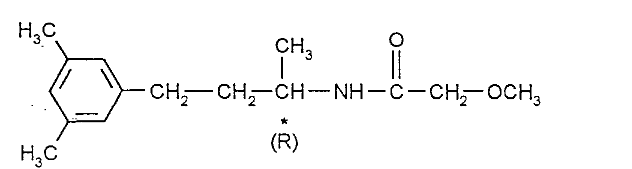

Als Säuren kommen bei der Durchführung der dritten Stufe des erfindungsgemäßen Verfahrens alle üblichen starken Säuren in Betracht. Vorzugsweise verwendbar sind Mineralsäuren, wie Schwefelsäure oder Salzsäure.

Als Basen kommen bei der Durchführung der dritten Stufe des erfindungsgemäßen Verfahrens alle üblichen starken Basen in Betracht. Vorzugsweise verwendbar sind anorganische Basen, wie Natrium- oder Kaliumhydroxid.

Als Vedünnungsmittel kommen bei der Durchführung der dritten Stufe des erfindungsgemäßen Verfahrens alle für derartige Umsetzungen üblichen organischen Solventien sowie Wasser in Betracht. Vorzugsweise verwendbar sind Wasser oder Mischungen aus Wasser und organischen Solventien, wobei Gemische aus Wasser und Toluol beispielhaft genannt seien.

Die Temperaturen können bei der Durchführung der dritten Stufe des erfindungsgemäßen Verfahrens innerhalb eines größeren Bereiches variiert werden. Im allgemeinen arbeitet man bei Temperaturen zwischen 20 und 180°C, vorzugsweise zwischen 30 und 150°C.

Bei der Durchführung der dritten Stufe des erfindungsgemäßen Verfahrens arbeitet man im allgemeinen unter Atmosphärendruck. Es ist aber auch möglich, unter erhöhtem oder vermindertem Druck zu arbeiten.

Bei der Durchführung der dritten Stufe des erfindungsgemäßen Verfahrens setzt man auf 1 Mol an acyliertem (R)-Amin der Formel (III) im allgemeinen 1 bis 5 Äquivalente oder auch einen größeren Überschuß an Säure oder Base ein. Die Aufarbeitung erfolgt nach üblichen Methoden. Im allgemeinen geht man so vor, daß man das Reaktionsgemisch nach beendeter Spaltung und Neutralisation mit einem mit Wasser nur wenig mischbaren, organischen Solvens extrahiert, die vereinigten organischen Phasen trocknet und einengt. Das dabei erhaltene Produkt kann gegebenenfalls nach üblichen Methoden von eventuell noch vorhandenen Verunreinigungen befreit werden.

Die nach dem erfindungsgemäßen Verfahren herstellbaren Amine der Formel (I*) sind wertvolle Zwischenprodukte zur Herstellung von Pharmazeutika oder von Wirkstoffen mit insektiziden, fungiziden oder herbiziden Eigenschaften (vgl. EP-A 0 519 211, EP-A 0 453 137, EP-A 0 283 879, EP-A 0 264 217 und EP-A 0 341 475). So erhält man z.B. die fungizid wirksame Verbindung der Formel 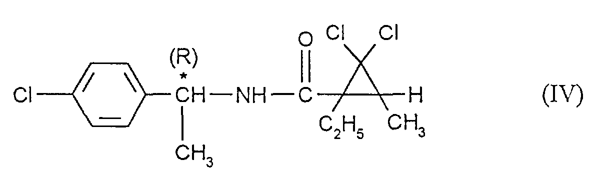 indem man das (R)-1-(4-Chlor-phenyl)-ethylamin der Formel 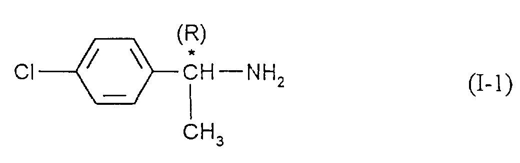 mit 2,2-Dichlor-1-ethyl-3-methyl-1-cyclopropancarbonsäurechlorid der Formel 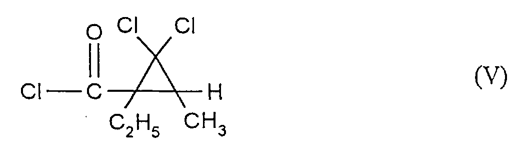 in Gegenwart eines Säurebindemittels und in Gegenwart eines inerten organischen Verdünnungsmittels umsetzt.

Die Durchführung des erfindungsgemäßen Verfahrens wird durch die folgenden Beispiele veranschaulicht.

### Herstellungsbeispiele

### Beispiel 1

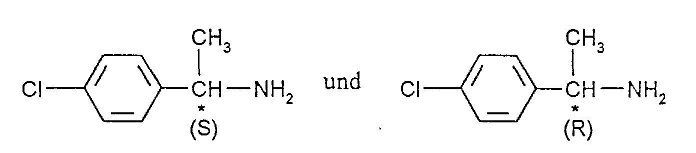

### 1. Stufe

Eine Lösung von 77,75 g (0,5 Mol) racemischem 1-(4-Chlorphenyl)-ethylamin in 260 g Methoxyessigsäuremethylester wird bei Raumtemperatur unter Rühren mit 3,9 g Novozym 435® (= immobilisierte Lipase aus Candida antarctica; 7300 U/g), versetzt. Man läßt noch weitere 2,5 Stunden bei Raumtemperatur rühren, filtriert dann das Enzym ab und wäscht mit 20 g Methoxyessigsäuremethylester nach.

### 2. Stufe

Das Filtrat wird unter vermindertem Druck eingeengt, und der verbleibende Rückstand wird in 300 ml Methylenchlorid aufgenommen. Die entstehende Lösung wird mit 100 ml Eiswasser und 21 ml konzentrierter Salzsäure versetzt und 0,2 Stunden bei Raumtemperatur gerührt. Danach werden die Phasen getrennt.

Die organische Phase wird nach dem Trocknen über Magnesiumsulfat unter vermindertem Druck eingeengt. Man erhält auf diese Weise 61,9 g eines Produktes, das gemäß gaschromatographischer Analyse zu 95 % aus dem (R)-Enantiomeren des Methoxyessigsäure-1-(4-chlorphenyl)-ethyl-amids besteht. Der ee-Wert beträgt 97,1 %.

Danach errechnet sich eine Ausbeute von 51,7 % der Theorie.

Die nach der obigen Phasentrennung erhaltene wäßrige Phase wird unter Kühlung mit 35 ml konzentrierter wäßriger Natronlauge versetzt und dann dreimal mit Methylenchlorid extrahiert. Die vereinigten organischen Phasen werden nach dem Trocknen über Magnesiumsulfat unter verminderten Druck eingeengt. Man erhält auf diese Weise 37 g einer farblosen Flüssigkeit, die gemäß gaschromatographischer Analyse zu 98,2 % aus dem (S)-Enantiomeren des 1-(4-Chlorphenyl)ethylamins besteht. Der ee-Wert beträgt 91,8 %.
Danach errechnet sich eine Ausbeute von 46,6 % der Theorie.

### 3. Stufe

Ein Gemisch aus 52 g (0,224 Mol) des erhaltenen (R)-Enantiomeren des Methoxyessigsäure-1-(4-chlor-phenyl)-ethylamids, 180 ml Wasser und 67,7 g konzentrierter Salzsäure wird 10 Stunden unter Rückfluß erhitzt. Nach dem Abkühlen auf Raumtemperatur wird das Reaktionsgemisch zunächst einmal mit 100 ml Methylenchlorid extrahiert, dann mit konzentrierter wäßriger Natronlauge alkalisch gestellt und anschließend dreimal mit je 200 ml Methylenchlorid extrahiert. Die vereinigten organischen Phasen werden über Magnesiumsulfat getrocknet und danach unter vermindertem Druck eingeengt. Man erhält auf diese Weise 33,1 g eines Produktes, das gemäß gaschromatographischer Analyse zu 99,1 % aus dem (R)-Enantiomeren des 1-(4-Chlor-phenyl)-ethylamins besteht. Der ee-Wert beträgt 95,1 %.

Es errechnet sich eine Gesamtausbeute von 95 % der Theorie.

### Beispiel 2

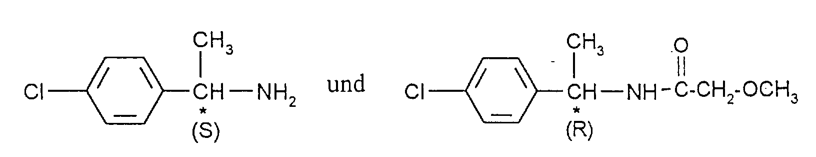

### 1. Stufe

Eine Lösung von 3,1 g (0,02 Mol) racemischem 1-(4-Chlorphenyl)-ethylamin in 20 ml tert.-Amyl-methyl-ether wird bei Raumtemperatur unter Rühren nacheinander mit 10,4 g (0,1 Mol) Methoxyessigsäure-methylester und 0,6 g Novozym 435® (= immobilisierte Lipase aus Candida antarctica; 7300 U/g) versetzt. Man rührt weiter bei 40°C und kontrolliert dabei das Fortschreiten der Reaktion, indem man Proben gaschromatographisch untersucht. Nach 1,5 Stunden ist ein Umsatz von 52 % erreicht. Zu diesem Zeitpunkt wird die Reaktion abgebrochen, indem man das Enzym abfiltriert.

### 2. Stufe

Das nach dem Abfiltrieren des Enzyms verbleibende Filtrat wird einer fraktionierten Destillation unterworfen.

Man erhält auf diese Weise 1,3 g einer farblosen Flüssigkeit, die gemäß gaschromatographischer Analyse zu 94 % aus dem (S)-Enantiomeren des 1-(4-Chlorphenyl)-ethylamins besteht. Der ee-Wert beträgt >98 %. Danach errechnet sich eine Ausbeute von 43 % der Theorie.

Außerdem erhält man 2,1 g eines Produktes, das gemäß gaschromatographischer Analyse zu 99 % aus dem (R)-Enantiomeren des Methoxyessigsäuie-1-(4-chlorphenyl)-ethyl-amids besteht. Der ee-Wert beträgt >98 %. Danach errechnet sich eine Ausbeute von 46,8 % der Theorie.

### Beispiel 3

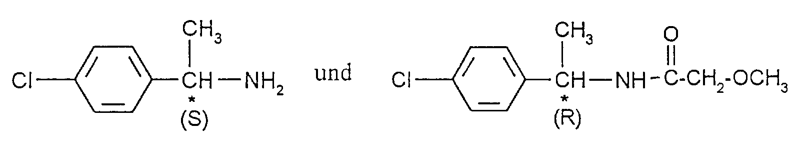

### 1. Stufe

Eine Lösung von 7,93 g (0,051 Mol) an racemischem 1-(4-Chlor-phenyl)-ethylamin in 40 ml Methyl-tert.-butylether wird bei Raumtemperatur unter Rühren nacheinander mit 13,2 g (0,1 Mol) Methoxyessigsäure-n-propylester und 0,45 g Novozym 435® (= immobilisierte Lipase aus Candida antarctica; 7300 U/g) versetzt. Man rührt weiter bei 40°C und kontrolliert das Fortschreiten der Reaktion, indem man Proben gaschromatographisch untersucht. Nach 1,5 Stunden ist ein Umsatz von 52 % erreicht. Zu diesem Zeitpunkt wird die Reaktion abgebrochen, indem man das Enzym abfiltriert.

### 2. Stufe

Das nach dem Abfiltrieren des Enzyms verbleibende Filtrat wird einer fraktionierten Destillation unterworfen.

Man erhält auf diese Weise 3,5 g (44,3 % der Theorie an dem (S)-Enantiomeren des 1-(4-Chlor-phenyl)-ethylamins. Der ee-Wert beträgt 96,4 %.

Außerdem erhält man 5,2 g (45,1 % der Theorie) an dem (R)-Enantiomeren des Methoxyessigsäure-1-(4-chlor-phenyl)-ethylamids. Der ee-Wert beträgt 99 %.

### Beispiel 4

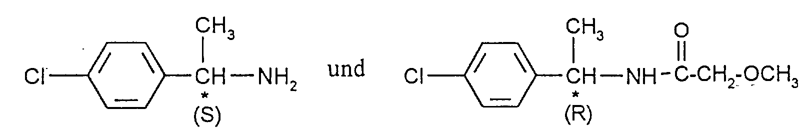

### 1. Stufe

Eine Lösung von 7,8 g (0,05 Mol) an racemischem 1-(4-Chlor-phenyl)-ethylamin in 35 ml Methyl-tert.-butyl-ether wird bei Raumtemperatur unter Rühren nacheinander mit 8,32 g (0,08 Mol) Methoxyessigsäure-methylester und 0,39 g Novozym 435® (= immobilisierte Lipase aus Candida antarctica; 7300 U/g) versetzt. Man rührt weiter bei Raumtemperatur und kontrolliert das Fortschreiten der Reaktion, indem man Proben gaschromatographisch untersucht. Nach 4,5 Stunden ist ein Umsatz von 55 % erreicht. Zu diesem Zeitpunkt wird die Reaktion abgebrochen, indem man das Enzym abfiltriert. In dem verbleibenden Filtrat weist das (S)-Enantiomere des 1-(4-Chlor-phenyl)-ethylamins einen ee-Wert von 98,1 % auf, während das (R)-Enantiomere des Methoxyessigsäure-1-(4-chlorphenyl)-ethylamids mit einem ee-Wert von 96 % anfällt.

### Beispiel 5

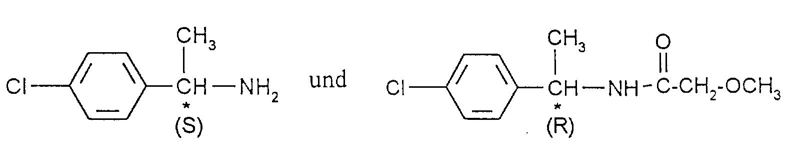

### 1. Stufe

Eine Lösung von 7,8 g (0,05 Mol) an racemischem 1-(4-Chlor-phenyl)-ethylamin in 40 ml Methyl-tert.-butyl-ether wird bei Raumtemperatur unter Rühren nacheinander mit 13,2 g (0,13 Mol) Methoxyessigsäure-methylester und 80 mg Novozym 435® (= immobilisierte Lipase aus Candida antarctica; 7300 U/g) versetzt. Man rührt weiter bei 40°C und kontrolliert das Fortschreiten der Reaktion, indem man Proben gaschromatographisch untersucht. Nach 3 Stunden ist ein Umsatz von 43,8 % erreicht. Zu diesem Zeitpunkt wird die Reaktion abgebrochen, indem man das Enzym abfiltriert. In dem verbleibenden Filtrat weist das (R)-Enantiomere des Methoxyessigsäure-1-(4-chlorphenyl)-ethylamids einen ee-Wert von 97,7 % auf.

### Beispiel 6

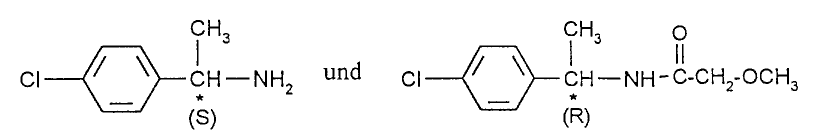

### 1. Stufe

Eine Lösung von 7,8 g (0,05 Mol) an racemischem 1-(4-Chlor-phenyl)-ethylamin in 40 ml Methyl-tert.-butyl-ether wird bei Raumtemperatur unter Rühren nacheinander mit 10,4 g (0,1 Mol) Methoxyessigsäure-methylester und 0,45 g Novozym 435® (= immobilisierte Lipase aus Candida antarctica; 7300 U/g) versetzt. Man rührt weiter bei 60°C und kontrolliert das Fortschreiten der Reaktion, indem man Proben gaschromatographisch untersucht. Nach 3 Stunden ist ein Umsatz von 54 % erreicht. Zu diesem Zeitpunkt wird die Reaktion abgebrochen, indem man das Enzym abfiltriert. In dem verbleibenden Filtrat weist das (S)-Enantiomere des 1-(4-Chlor-phenyl)-ethylamins einen ee-Wert von 98,8 % auf, während das (R)-Enantiomere des Methoxyessigsäure-1-(4-chlorphenyl)-ethylamids mit einem ee-Wert von 94,4 % anfällt.

### Beispiel 7

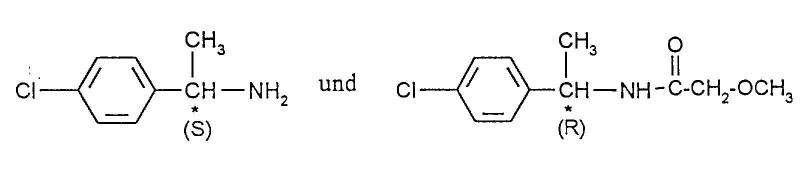

### 1. Stufe

Eine Lösung von 16,6 g (0,107 Mol) an racemischem 1-(4-Chlor-phenyl)-ethylamin in 41 g Methoxyessigsäure-methylester wird unter Rühren bei Raumtemperatur mit 1,2 g Novozym 435® (= immobilisierte Lipase aus Candida antarctica; 7300 U/g) versetzt. Man rührt weiter bei 40°C und kontrolliert das Fortschreiten der Reaktion, indem man Proben gaschromatographisch untersucht. Nach 2,5 Stunden ist ein Umsatz von 54 % erreicht. Zu diesem Zeitpunkt wird die Reaktion abgebrochen, indem man das Enzym abfiltriert. In dem verbleibenden Filtrat weist das (S)-Enantiomere des 1-(4-Chlor-phenyl)-ethylamins einen ee-Wert von 95,6 % auf, während das (R)-Enantiomere des Methoxyessigsäure-1-(4-chlorphenyl)-ethylamids mit einem ee-Wert von 95,3 % anfällt.

### Beispiel 8

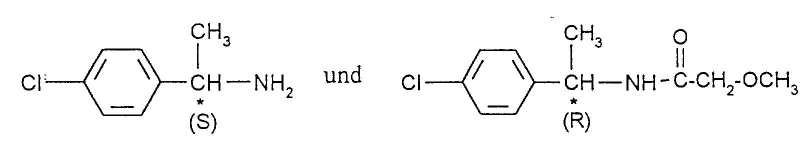

### 1. Stufe

Eine Lösung von 20,06 g (0,129 Mol) an racemischem 1-(4-Chlor-phenyl)-ethylamin in 20 g (0,19 Mol) Methoxyessigsäuremethylester wird unter Rühren bei Raumtemperatur mit 1 g Novozym 435® (= immobilisierte Lipase aus Candida antarctica; 7300 U/g) versetzt. Man rührt noch eine Stunde bei Raumtemperatur weiter und bricht dann die Reaktion ab, indem man das Enzym abfiltriert. Der Umsatz beträgt 43,9 %. In dem verbleibenden Filtrat weist das (S)-Enantiomere des 1-(4-Chlor-phenyl)-ethylamins einen ee-Wert von 61 % auf, während das (R)-Enantiomere des Methoxyessigsäure-1-(4-chlorphenyl)-ethylamids mit einem ee-Wert von 98,4 % anfällt.

### Beispiel 9

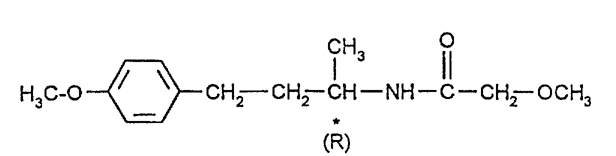

Ein Gemisch aus 17,9 g (0,1 mol) an racemischem 1-Methyl-3-(4-methoxy-phenyl)-propylamin, 10,4 g (0,1 mol) Methoxyessigsäure-methylester, 0,75 g Novozym 435® (immobilisierte Lipase aus Candida antarctica) und 80 ml Methyl-tert.-butylether wird 3 Stunden bei 40°C gerührt. Danach wird das Enzym abfiltriert. Die verbleibende Lösung wird mit 100 ml 10 %iger wäßriger Salzsäure versetzt und dann unter vermindertem Druck eingeengt. Der Rückstand wird auf 0°C abgekühlt, und der dabei anfallende Feststoff wird abfiltriert und getrocknet.
Man erhält auf diese Weise 9,95 g eines Produktes, das gemäß Gaschromatogramm zu 99,2 % aus dem (R)-Enantiomeren des Methoxyessigsäure-[3-(4-methoxy-phenyl)-1-methyl]-propylamids besteht. Die Ausbeute errechnet sich danach zu 39,6 % der Theorie.
Der ee-Wert beträgt 99 %.
¹H-NMR-Spektrum / CDCl₃ / TMS):
- δ =: 1,1 (d, 3H, CH₃); 1,63-1,71 (m, 2H, CH₂); 2,48-2,58 (m, 2H, CH₂); 3,32 (s, 3H, CH₃); 3,69 (s, 3H, CH₃); 3,78 (d, 2H,CH₂); 4,0 (m, H, CH); 6,3 (d, 1H, NH); 6,7-7,03 (m, 4H, aromat. H) ppm

### Beispiel 10

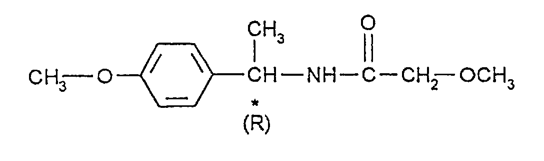

Ein Gemisch aus 51,3 g (0,34 mol) an racemischem 1-(4-Methoxy-phenyl)-ethylamin, 35,3 g (0,34 mol) Methoxyessigsäure-methylester, 2,5 g Novozym 435® (= immobilisierte Lipase aus Candida antarctica) und 80 ml Methyl-tert.-butylether wird 5 Stunden bei 40°C gerührt. Danach wird das Enzym abfiltriert. Die verbleibende Lösung wird mit 100 ml 10 %iger wäßriger Salzsäure versetzt und dann unter vermindertem Druck eingeengt. Der Rückstand wird auf 0°C abgekühlt, und der dabei anfallende Feststoff wird abfiltriert und getrocknet. Man erhält auf diese Weise 38,5 g eines Produktes, das gemäß Gaschromatogramm zu 96 % aus dem (R)-Enantiomeren des Methoxyessigsäure-1-(4-methoxy-phenyl)-ethylamids besteht. Die Ausbeute errechnet sich danach zu 40,2 % der Theorie.
Der ee-Wert beträgt 98,3 %.
¹H-NMR-Spektrum */* CDCl₃ / TMS):
- δ =: 1,49 (d, 3H, CH₃); 3,38 (s, 3H, CH₃); 3,79 (s, 3H, CH₃); 3,87 (d, 2H, CH₂); 5,13 (m, 1H, CH); 6,86 (d, 1H, NH); 6,9-7,28 (m, 4H, aromat. H) ppm.

## Patentansprüche

1. Verfahren zur Herstellung von optisch aktiven Aminen der Formel 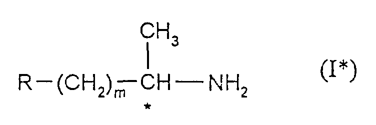 in welcher
R für substituiertes Phenyl der Formel 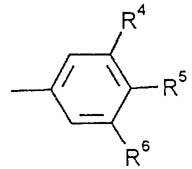 steht, worin
R⁴, R⁵ und R⁶ unabhängig voneinander für Wasserstoff, Fluor, Chlor, Brom, Methyl, Ethyl, n-Propyl, Isopropyl, n-Butyl, iso-Butyl, sek.-Butyl, Methoxy, Ethoxy, Methylthio, Trichlormethyl, Trifluormethyl, Difluormethyl, Trifluormethoxy, Difluorchlormethoxy, Difluormethoxy, Cyano, Dimethylamino, Diethylamino, Nitro, Phenyl, Phenoxy oder Benzyl stehen,
wobei aber mindestens einer der Reste
R⁴, R⁵ und R⁶ nicht für Wasserstoff steht,
oder
R für gegebenenfalls einfach bis dreifach, gleichartig oder verschieden durch Fluor, Chlor, Brom, Methyl, Ethyl, n-Propyl, Isopropyl, n-Butyl, iso-Butyl, sek.-Butyl, Methoxy, Ethoxy, Trichlormethyl, Trifluormethyl, Difluormethyl, Trifluormethoxy, Difluorchlormethoxy und/oder Difluormethoxy substituiertes Naphthyl steht, wobei aber die ortho-Positionen zu dem Kohlenstoffatom, über das der Naphthyl-Rest gebunden ist, nicht substituiert sind,
oder
R für gegebenenfalls benzanelliertes Furyl, Thienyl, Pyridyl oder Pyrimidin steht, wobei diese Reste einfach bis dreifach, gleichartig oder verschieden substituiert sein können durch Fluor, Chlor, Brom, Methyl, Ethyl, n-Propyl, Isopropyl, n-Butyl, iso-Butyl, sek.-Butyl, tert.-Butyl, Methoxy, Ethoxy, n-Propoxy, iso-Propoxy, Trifluormethyl und/oder Trifluorethyl, wobei aber die zur Verknüpfungsstelle benachbarten Positionen der Heteroarylgruppe keine Substituenten tragen,
oder
R für geradkettiges oder verzweigtes Alkyl mit 1 bis 5 Kohlenstoffatomen oder Halogenalkyl mit 1 bis 5 Kohlenstoffatomen und 1 bis 3 Fluor- und/oder Chloratomen steht,
und
m für die Zahlen 0, 1 oder 2 steht,
**dadurch gekennzeichnet, daß** man
a) in einer ersten Stufe racemische Amine der Formel 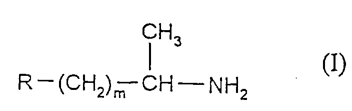 in welcher
R und m die oben angegebenen Bedeutungen haben,
mit Estern der Formel 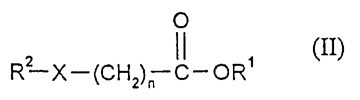 in welcher
R¹ für Methyl, Ethyl, n-Propyl, n-Butyl, Chlormethyl, 2-Chlorethyl, 2-Fluorethyl oder 2,2,2-Trifluorethyl steht,
R² für Wasserstoff, Methyl, Ethyl, n-Propyl, n-Butyl, Chlormethyl, Trifluormethyl, 2-Chlorethyl oder für gegebenenfalls einfach oder zweifach durch Fluor, Chlor, Brom, Amino, Hydroxy, Methyl, Ethyl, Methoxy, Phenyl und/oder Phenoxy substituiertes Phenyl steht,
X für Sauerstoff oder Schwefel steht und
n für die Zahlen 1 oder 2 steht;
in Gegenwart von Lipase aus Candida antarctica sowie gegebenenfalls in Gegenwart eines Verdünnungsmittels umsetzt,
b) in einer zweiten Stufe das erhaltene Gemisch aus (S)-Amin der Formel 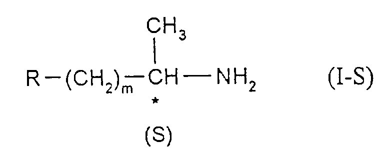 in welcher
R und m die oben angegebenen Bedeutungen haben,
und acyliertem (R)-Amin der Formel 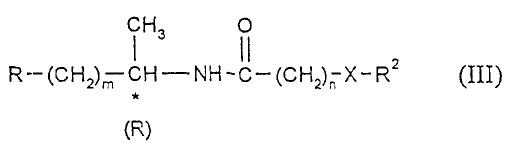 in welcher
R, R², X, m und n die oben angegebenen Bedeutungen haben,
trennt und
c) gegebenenfalls in einer dritten Stufe aus acyliertem (R)-Amin der Formel (III) durch Behandlung mit Säure oder Base, gegebenenfalls in Gegenwart eines Verdünnungsmittels (R)-Amin der Formel 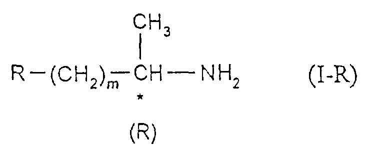 in welcher
R und m die oben angegebenen Bedeutungen haben,
freisetzt.

2. Verfahren gemäß Anspruch 1, **dadurch gekennzeichnet, daß** man als racemisches Amin der Formel (I) das 1-(4-Chlorphenyl)-ethylamin der Formel 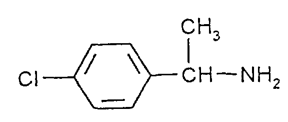 einsetzt.

3. Verfahren gemäß Anspruch 1, **dadurch gekennzeichnet, daß** man als Ester der Formel (II) den Methoxyessigsäuremethylester der Formel 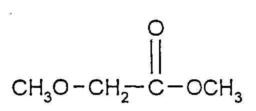 einsetzt.

4. Verfahren gemäß Anspruch 1, **dadurch gekennzeichnet, daß** man bei der Durchführung der ersten Stufe bei Temperaturen zwischen 0°C und 80°C arbeitet.

5. Acylierte (R)-Amine der Formel 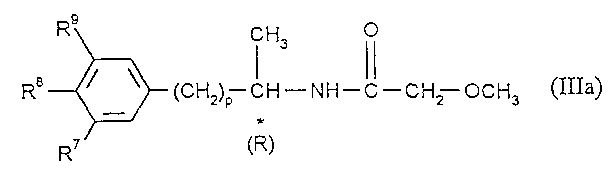 in welcher
R⁸ für Fluor, Chlor, Brom, Methyl, Methoxy oder Methylthio steht,
R⁷ und R⁹ für Wasserstoff stehen und
p für die Zahlen 0, 1 oder 2 steht,
oder
R⁸ für Wasserstoff steht,
R⁷ und R⁹ für Methyl stehen und
p für 2 steht.

## Claims

1. Process for preparing optically active amines of the formula 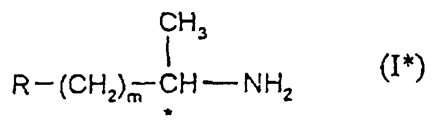 in which
R represents substituted phenyl of the formula 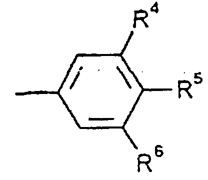 in which
R⁴, R⁵ and R⁶ independently of one another represent hydrogen, fluorine, chlorine, bromine, methyl, ethyl, n-propyl, isopropyl, n-butyl, iso-butyl, sec-butyl, methoxy, ethoxy, methylthio, trichloromethyl, trifluoromethyl, difluoromethyl, trifluoromethoxy, difluorochloromethoxy, difluoromethoxy, cyano, dimethylamino, diethylamino, nitro, phenyl, phenoxy or benzyl,
but where at least one of the radicals
R⁴, R⁵ and R⁶ does not represent hydrogen,
or
R represents naphthyl which is optionally mono- to trisubstituted by identical or different substituents from the group consisting of fluorine, chlorine, bromine, methyl, ethyl, n-propyl, isopropyl, n-butyl, iso-butyl, sec-butyl, methoxy, ethoxy, trichloromethyl, trifluoromethyl, difluoromethyl, trifluoromethoxy, difluorochloromethoxy and difluoromethoxy, but where the positions ortho to the carbon atom via which the naphthyl radical is attached are not substituted,
or
R represents optionally benzo-fused furyl, thienyl, pyridyl or pyrimidine, where these radicals may be mono- to trisubstituted by identical or different substituents from the group consisting of fluorine, chlorine, bromine, methyl, ethyl, n-propyl, isopropyl, n-butyl, iso-butyl, sec-butyl, tert-butyl, methoxy, ethoxy, n-propoxy, iso-propoxy, trifluoromethyl and/or trifluoroethyl, but where the positions of the heteroaryl group which are adjacent to the point of attachment do not carry any substituents,
or
R represents straight-chain or branched alkyl having 1 to 5 carbon atoms or halogenoalkyl having 1 to 5 carbon atoms and 1 to 3 fluorine and/or chlorine atoms,
and
m represents the numbers 0, 1 or 2,
**characterized in that**
a) in a first step, the racemic amines of the formula 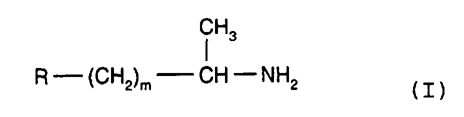 in which
R and m are each as defined above
are reacted with esters of the formula 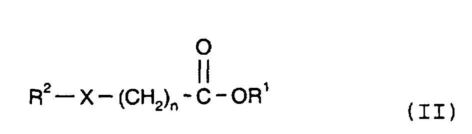 in which
R¹ represents methyl, ethyl, n-propyl, n-butyl, chloromethyl, 2-chloroethyl, 2-fluoroethyl or 2,2,2-trifluoroethyl,
R² represents hydrogen, methyl, ethyl, n-propyl, n-butyl, chloromethyl, trifluoromethyl, 2-chloroethyl or represents phenyl which is optionally mono- or disubstituted by fluorine, chlorine, bromine, amino, hydroxyl, methyl, ethyl, methoxy, phenyl and/or phenoxy,
X represents oxygen or sulphur and
n represents the numbers 1 or 2,
in the presence of lipase from Candida antarctica and, if appropriate, in the presence of a diluent,
b) in a second step, the resulting mixture of (S)-amine of the formula 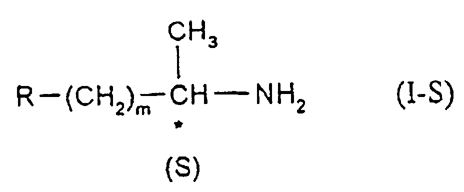 in which
R and m are each as defined above,
and acylated (R)-amine of the formula 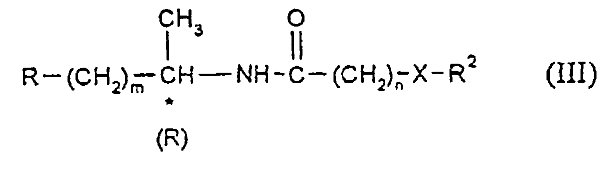 in which
R, R², X, m and n are each as defined above,
is separated and
c) if appropriate, in a third step, the (R)-amine of the formula 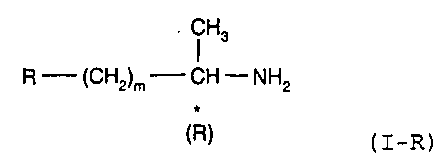 in which
R and m are each as defined above
is set free from acylated (R)-amine of the formula (III) by treatment with acid or base, if appropriate in the presence of a diluent.

2. Process according to Claim 1, **characterized in that** the racemic amine of the formula (I) used is 1-(4-chlorophenyl)-ethylamine of the formula

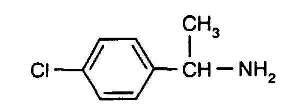

3. Process according to Claim 1, **characterized in that** the ester of the formula (II) used is methyl methoxyacetate of the formula

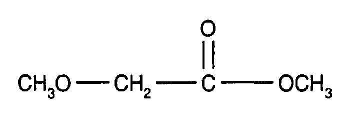

4. Process according to Claim 1, **characterized in that** the first step is carried out at temperatures between 0°C and 80°C.

5. Acylated (R)-amines of the formula 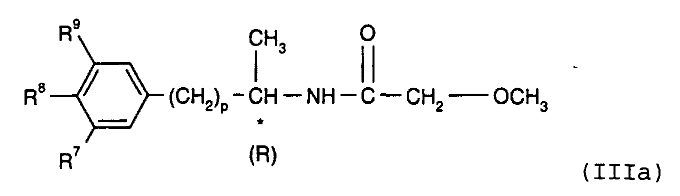 in which
R⁸ represents fluorine, chlorine, bromine, methyl, methoxy or methylthio,
R⁷ and R⁹ each represent hydrogen and
p represents the numbers 0, 1 or 2,
or
R⁸ represents hydrogen,
R⁷ and R⁹ each represent methyl and
p represents 2.

## Revendications

1. Procédé pour la préparation d'amines optiquement actives répondant à la formule 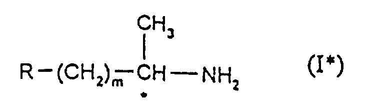 dans laquelle
R représente un groupe phényle substitué répondant à la formule 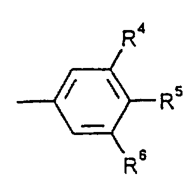 dans laquelle
R⁴, R⁵ et R⁶ représentent, indépendamment l'un de l'autre un atome d'hydrogène, un atome de fluor, un atome de chlore, un atome de brome, un groupe méthyle, un groupe éthyle, un groupe n-propyle, un groupe isopropyle, un groupe n-butyle, un groupe isobutyle, un groupe sec.-butyle, un groupe méthoxy, un groupe éthoxy, un groupe méthylthio, un groupe trichlorométhyle, un groupe trifluorométhyle, un groupe difluorométhyle, un groupe trifluorométhoxy, un groupe difluorochlorométhoxy, un groupe difluorométhoxy, un groupe cyano, un groupe diméthylamino, un groupe diéthylamino, un groupe nitro, un groupe phényle, un groupe phénoxy ou un groupe benzyle;
dans lesquels toutefois au moins un des radicaux R⁴, R⁵ et R⁶ ne représente pas un atome d'hydrogène;
ou
R représente un radical naphtyle portant le cas échéant de un à trois substituants identiques ou différents, fluoro, chloro, bromo, méthyle, éthyle, n-propyle, isopropyle, n-butyle, isobutyle, sec.-butyle, méthoxy, éthoxy, trichlorométhyle, trifluorométhyle, difluorométhyle, trifluorométhoxy, difluorochiorométhoxy et/ou difluorométhoxy, dans lequel toutefois les positions ortho par rapport à l'atome de carbone par lequel est lié le radical naphtyle ne sont pas substituées;
ou
R représente un radical furyle, un radical thiényle, un radical pyridyle ou un radical pyrimidine, le cas échéant benzocondensé, ces radicaux pouvant porter de 1 à 3 substituants identiques ou différents fluoro, chloro, bromo, méthyle, éthyle, n-propyle, isopropyle, n-butyle, isobutyle, sec.-butyle, tert.-butyle, méthoxy, éthoxy, n-propoxy, isopropoxy, trifluorométhyle et/ou trifluoréthyle, dans lequel toutefois les positions du groupe hétéroaryle voisines de l'endroit de liaison ne portent pas de substituant,
ou
R représente un groupe alkyle à chaîne droite ou ramifiée contenant de 1 à 5 atomes de carbone ou un groupe halogénalkyle contenant de 1 à 5 atomes de carbone et de 1 à 3 atomes de fluor et/ou de chlore,
et
m représente les nombres 0, 1 ou 2,
**caractérisé en ce que**
a) dans une première étape, on fait réagir des amines racémiques répondant à la formule 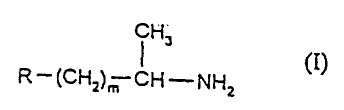 dans laquelle
R et m ont les significations indiquées ci-dessus,
avec des esters répondant à la formule 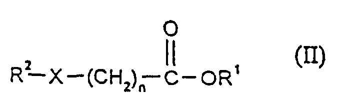 dans laquelle
R¹ représente un groupe méthyle, un groupe éthyle, un groupe n-propyle, un groupe n-butyle, un groupe chlorométhyle, un groupe 2-chloréthyle, un groupe 2-fluoréthyle ou un groupe 2,2,2-trifluoréthyle,
R² représente un atome d'hydrogène, un groupe méthyle, un groupe éthyle, un groupe n-propyle, un groupe n-butyle, un groupe chlorométhyle, un groupe trifluorométhyle, un groupe 2-chloréthyle ou encore un groupe phényle portant, le cas échéant, 1 ou 2 substituants identiques ou différents, fluoro, chloro, bromo, amino, hydroxyle, méthyle, éthyle, méthoxy, phényle et/ou phénoxy,
X représente un atome d'oxygène ou un atome de soufre, et
n représente les nombres 1 ou 2,
en présence de lipase provenant de Candida antarctica et, le cas échéant, en présence d'un diluant,
b) dans une deuxième étape, on sépare le mélange obtenu constitué par la (S)-amine répondant à la formule 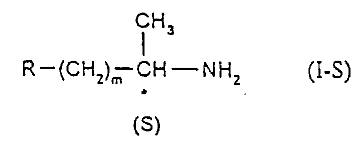 dans laquelle
R et m ont les significations indiquées ci-dessus,
et par la (R)-amine acylée répondant à la formule 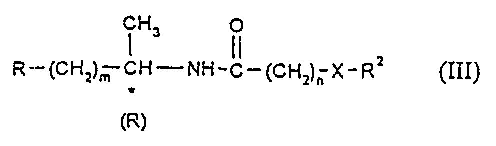 dans laquelle
R, R², X, m et n ont les significations indiquées ci-dessus,
et
c) le cas échéant, dans une troisième étape, on libère de la (R)-amine acylée répondant à la formule (III), par traitement avec un acide ou avec une base, le cas échéant, en présence d'un diluant, la (R)-amine répondant à la formule 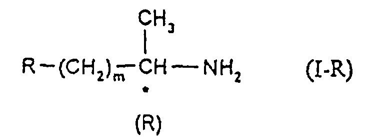 dans laquelle
R et m ont les significations indiquées ci-dessus.

2. Procédé selon la revendication 1, **caractérisé en ce qu'**on met en oeuvre, à titre d'amine racémique répondant à la formule (I), la 1-(4-chlorophényl)-éthylamine répondant à la formule

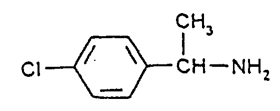

3. Procédé selon la revendication 1, **caractérisé en ce qu'**on met en oeuvre, à titre d'ester répondant à la formule (II), l'ester méthylique de l'acide méthoxyacétique répondant à la formule

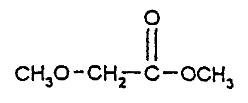

4. Procédé selon la revendication 1, **caractérisé en ce qu'**on travaille, dans la mise en oeuvre de la première étape, à des températures entre 0°C et 80°C.

5. (R)-amines acylées répondant à la formule 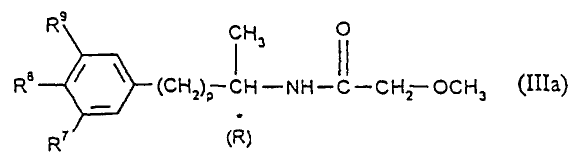 dans laquelle
R⁸ représente un atome de fluor, un atome de chlore, un atome de brome, un groupe méthyle, un groupe méthoxy ou un groupe méthylthio,
R⁷ et R⁹ représentent un atome d'hydrogène, et
p représente les nombres 0, 1 ou 2,
ou
R⁸ représente un atome d'hydrogène,
R⁷ et R⁹ représentent un groupe méthyle, et
p est égal à 2.
